# EUROPEAN PATENT APPLICATION

(11) **EP 3 424 488 A1**
(43) Date of publication of application: **09.01.2019**
(21) Application number: 17759985.9
(22) Date of filing: 28.02.2017
(51) Int. Cl.: A61K 8/891, A61K 8/34, A61K 8/86, A61Q 1/02, A61Q 19/00

(54) **AQUEOUS COSMETIC COMPOSITION**

(30) Priority: 29.02.2016 JP 2016038176
(71) Applicant: Shiseido Co., Ltd., Tokyo 104-8010 (JP)
(72) Inventor: ASAI, Ayumi, Yokohama-shi Kanagawa 224-8558 (JP); ONO, Akinori, Yokohama-shi Kanagawa 224-8558 (JP)
(74) Representative: Ter Meer Steinmeister & Partner
(86) International application number: PCT/JP2017/007750
(87) International publication number: WO 2017/150519

(57) **Abstract**

The purpose of the present invention is to provide an aqueous cosmetic composition, in which a proper amount of a hydrophobic spherical powder can be blended in an aqueous phase stably even though an aqueous base material having a fresh sensation upon use is used, and which can correct the appearance of skin naturally. The present invention relates to a cosmetic composition characterized by comprising (A) at least one higher alcohol having a saturated straight alkyl chain, (B) hydrophilic surfactants including a nonionic surfactant, (C) a powder having an average particle diameter of 1 to 30 µm in an amount of 4 to 30% by mass and (D) water, wherein the ratio of the number of moles of a hydrophilic nonionic surfactant to the total number of moles of the hydrophilic surfactants (B) is 0.5 or more, the percentage of the amount of a hydrophobic spherical powder in the total amount of the powder having an average particle diameter of 1 to 30 µm (C) is 50% by mass or more, and the hydrophobic spherical powder is added to an aqueous phase.

## Description

### Technical Field

The present invention relates to an aqueous cosmetic composition stably incorporating a hydrophobic spherical powder. More specifically, the invention relates to an aqueous cosmetic composition in which a hydrophobic spherical powder is stably incorporated in an aqueous base, the composition having a fresh feeling of use and capable of correcting the skin appearance naturally.

### Background Art

One of the important roles required for makeup cosmetics is an "aesthetic role" of making the appearance look beautiful. In particular, the "aesthetic role" of cosmetics that are applied to the skin, such as creams and foundations, encompasses adjustment of the color tone of the skin and correction of irregularities due to, for example, skin pores. Conventional foundations and the like incorporate components having high refractive indices for correcting the color tone of the skin and powdery components having a light diffusing effect for making the irregularities of the skin inconspicuous.

For example, Patent Document 1 discloses a water-in-oil type cosmetic for pore-masking use. The cosmetic incorporates glycylglycine suppressing parakeratosis due to sebum as one factor causing conspicuous pores stably with a crosslinked polyether-modified organopolysiloxane and further incorporates titanium dioxide having a pore concealing effect and having an average particle size of 0.2 to 0.5 µm or a composite powder having the titanium dioxide as a nucleus.

Patent Document 2 describes a cosmetic for correction of irregularities incorporating a solid wax, an oil gelling agent, an elastic powder selected from a group consisting of silicone resin powder, a urethane resin powder, and a polyamide resin powder, and a nonvolatile oil at a predetermined ratio, and it is stated that large irregularities on the skin can be corrected by forming a thick coating film.

As described above, most of known cosmetics having an effect of correcting irregularities are oily cosmetics or water-in-oil emulsion cosmetics. In such a case, when the cosmetic is applied to the skin, since the oil phase is first brought into contact with the skin, a relatively strong oily feeling cannot be denied. In addition, due to a large amount of the oil, sticky feeling may occur after application.

In contrast, oil-in-water emulsion cosmetics provide a fresh feeling at the application. However, most of the components for correcting the skin appearance incorporated in known cosmetics are hydrophobic powders, and therefore, it is necessary to increase the amounts of an oil and a surfactant for incorporating the hydrophobic powder in an amount for achieving a sufficient correcting effect, resulting in occurrence of a sticky feeling in some cases.

Patent Document 3 describes an oil-in-water emulsion cosmetic having excellent water resistance, containing α-monoacryl glyceryl ether, a wax, a silicone oil, and a silicone resin at a specific ratio, and incorporating a hydrophobized powder. However, even in this cosmetic, since the hydrophobized powder is incorporated in the oil phase, it is necessary to increase the amounts of the oil and the surfactant in order to increase the amount of the hydrophobic powder for improving the correction effect on the skin appearance.

The surface of the skin is considered to be hydrophobic. For example, it has been confirmed that when a powder conventionally incorporated in the oil phase is replaced with a hydrophilic powder to incorporate a large amount of the hydrophilic powder in the aqueous phase, the affinity to the skin decreases and a powdery texture is given.

### Citation List

### Patent Document

Patent Document 1: JP-A 2009-155274
Patent Document 2: JP-A 2010-30971
Patent Document 3: JP-B 4278313

### Disclosure of Invention

### Technical Problem

The present invention has been made in view of the above-described conventional art, and it is a problem to be solved is providing an aqueous cosmetic composition in which an appropriate amount of a hydrophobic spherical powder can be stably incorporated in an aqueous phase in order to make possible to correct the skin appearance naturally, although it contains an aqueous base having a fresh feeling of use.

### Solution to Problem

The present inventors have earnestly studied to solve the above-mentioned problem and consequently have found that a hydrophobic spherical powder in an amount effective for correcting the skin appearance can be stably incorporated in an aqueous cosmetic containing (A) a higher alcohol, (B) a hydrophilic surfactant, (C) 4 to 30 % by mass of a powder having an average particle size of 1 to 30 µm, and (D) water by designing the composition of the (B) hydrophilic surfactant and the composition of the (C) powder, and the present invention has been accomplished.

That is, the present invention provides a cosmetic composition comprising:
(A) one or more of higher alcohol having a saturated straight-chain alkyl chain;
(B) a hydrophilic surfactant including a nonionic surfactant;
(C) 4 to 30 % by mass of a powder having an average particle size of 1 to 30 µm; and
(D) water,
wherein a ratio of the number of moles of the hydrophilic nonionic surfactant to the total number of moles of the (B) hydrophilic surfactant is 0.5 or more; and a percentage of the content of a hydrophobic spherical powder in the total content of the (C) powder having an average particle size of 1 to 30 µm is 50 % by mass or more, where the hydrophobic spherical powder is contained in an aqueous phase.

### Advantageous Effects of Invention

The cosmetic composition of the present invention stably incorporates a hydrophobic spherical powder and thereby has an excellent correction effect on skin appearance while providing a fresh feeling of use like that of known skin care cosmetics. Unlike conventional oily foundations, the cosmetic composition of the present invention does not give users feelings of burdens such as powdery texture or oily and sticky feeling and can be provided as a new-type makeup cosmetic that can correct the skin appearance naturally. That is, the aqueous cosmetic composition of the present invention is applicable as a skin care cosmetic and also as a makeup cosmetic.

### Description of Embodiments

The cosmetic composition of the present invention is an "aqueous cosmetic composition". Throughout the present specification, the term "aqueous cosmetic composition" refers to an oil-free cosmetic or an oil-in-water emulsion cosmetic containing 30 % by mass or less of oil.

The cosmetic composition of the present invention contains, as essential components, (A) one or more of higher alcohols having a saturated straight-chain alkyl chain, (B) a hydrophilic surfactant, (C) 4 to 30 % by mass of a powder having an average particle size of 1 to 30 µm, and (D) water.
Each component will now be described in detail.

### (A) Higher alcohol

The component (A) in the present invention is composed of one or more higher alcohols having a saturated straight-chain alkyl chain. The higher alcohol having a saturated straight-chain alkyl chain is an alcohol having a saturated straight-chain alkyl (linear saturated alkyl) group having 12 or more, preferably 12 to 22 carbon atoms.

Examples of the higher alcohol used in the present invention include lauryl alcohol (dodecanol), tridecanol, myristyl alcohol (tetradecanol), pentadecanol, cetyl alcohol (hexadecanol), heptadecanol, stearyl alcohol (octadecanol), nonadexanol, and behenyl alcohol (docosanol). In the present invention, these higher alcohols may be incorporated alone or in combination of two or more thereof.

Although the content of the (A) higher alcohol having a saturated straight-chain alkyl chain in the cosmetic composition of the present invention is not particularly limited, the content is 0.1 to 8 % by mass and preferably 0.3 to 6 % by mass.

### (B) Hydrophilic surfactant

The component (B), hydrophilic surfactant, in the present invention must include a nonionic surfactant as an essential component and may optionally include an ionic surfactant.

The nonionic surfactant (hereinafter may be referred to as "hydrophilic nonionic surfactant") constituting the (B) hydrophilic surfactant of the present invention can be selected from nonionic surfactants having an HLB of 10 or more that have been used conventionally in cosmetics and the like. The HLB is a concept indicating the hydrophilicity and hydrophobicity of a surfactant and is generally indicated by a value of 0 to 20, from the most hydrophobic (HLB = 0) to the most hydrophilic (HLB = 20). The hydrophilic nonionic surfactant in the present invention is required to have an HLB value of 10 or more, and a nonionic surfactant having an HLB value of 11 or more, 12 or more, 13 or more, or 14 or more is preferably used.

Examples of the hydrophilic nonionic surfactant used in the present invention include polyoxyethylene fatty acid glyceryls, polyoxyethylene/methylpolysiloxane copolymers, fatty acid polyoxyethylene sorbitan, polyoxyethylene alkyl ethers, maltitol hydroxy aliphatic alkyl ethers, alkylated polysaccharides, alkyl glucosides, sucrose fatty acid esters, and polyoxyethylene hydrogenated castor oil glyceryls.

Among the hydrophilic nonionic surfactants, ethers of a saturated straight-chain alkyl chain (preferably having 12 to 22 carbon atoms) and a polyalkylene glycol (e.g., polyethylene oxide, polypropylene oxide, or polybutylene oxide) are preferably used. Examples of the ether include, but not limited to, POE (20) cetyl ether, POE (30) cetyl ether, POE (20) stearyl ether, POE (25) stearyl ether, POE (30) stearyl ether, POE (20) behenyl ether, POE (30) behenyl ether, POE (60) glycerin monoisostearate, POE (20) sorbitan monostearate, coconut fatty acid sorbitan, coconut oil fatty acid PEG-7 glyceryl, lauramide MEA, lauryl pyridinium chloride, PEG-32 laurate, PEG-40 hydrogenated castor oil laurate, PEG-14 oleate, polyglyceryl-6 oleate, PEG-32 dilaurate, and PEG-11 cocamide. In particular, it is preferred to use one or more selected from POE (20) cetyl ether, POE (30) cetyl ether, POE (20) stearyl ether, POE (30) stearyl ether, and POE (30) behenyl ether.

The ionic surfactant used in the present invention can be selected from anionic, cationic, and amphoteric surfactants that have been used conventionally in cosmetics and the like, and examples thereof include the followings.

Examples of the ionic surfactant include fatty acids having 12 to 22 carbon atoms and salts thereof, such as palmitic acid, stearic acid, and isostearic acid; N-acyl sarcosine having 12 to 22 carbon atoms and salts thereof, such as sodium lauroyl sarcosine; alkyl phosphoric acid having 12 to 22 carbon atoms and salts thereof, such as sodium cetyl phosphate; acyl methyl taurate having 12 to 22 carbon atoms and salts thereof, such as sodium stearoyl methyl taurate; acyl lactic acid having 12 to 22 carbon atoms and salts thereof, such as sodium stearoyl lactylate; acyl glutamate having 12 to 22 carbon atoms and salts thereof, such as sodium stearoyl glutamate; distearyl dimethyl ammonium chloride; alkyl trimethyl ammonium chloride; benzalkonium chloride; and alkyl dimethyl aminoacetic acid betaine.

In the cosmetic composition of the present invention, the ratio (X) of the number of moles of the hydrophilic nonionic surfactant (B1) to the total number of moles of the hydrophilic surfactant (B) (i.e., the sum of the number of moles of the hydrophilic nonionic surfactant (B1) and the number of moles of the ionic surfactant (B2)), X = B1/(B1+B2), is required to be 0.5 or more. The molar ratio (X) is preferably 0.65 or more and more preferably 0.8 or more, and the (B) hydrophilic surfactant may include only the hydrophilic nonionic surfactant (i.e., molar ratio (X) may be 1). If the molar ratio is less than 0.5, the stability of the composition is reduced, and the uniformity of appearance when applied to the skin is also reduced.

Although the content of the (B) hydrophilic surfactant (the sum of the content of the nonionic surfactant and the content of the ionic surfactant) in the cosmetic composition of the present invention is not particularly limited, the content is usually 0.05 to 6 % by mass and preferably 0.1 to 5 % by mass.

### (C) Powder having an average particle size of 1 to 30 µm

The component (C) in the cosmetic composition of the present invention is a powder having an average particle size of 1 to 30 µm. The average particle size can be any value within the range of 1 to 30 µm. For example, the lower limit of the average particle size range can be, for example, 1 µm, 1.5 µm, or 2 µm, and the upper limit can be 30 µm, 25 µm, 20 µm, 18 µm, or 15 µm.
The term "average particle size" in the present specification refers to an average value of particle sizes determined by laser analysis.

In the cosmetic composition of the present invention, the percentage (Y) of the content of the (C1) hydrophobic spherical powder to the total content of the (C) powder having an average particle size of 1 to 30 µm (the sum of the content of the (C1) hydrophobic spherical powder and the content of (C2) powders other than the hydrophobic spherical powder), Y=[C1/(C1+C2)]x100, is required to be 50 % by mass or more. The percentage (Y) is preferably 60 % by mass or more and more preferably 70 % by mass or more, and the component (C) may include only the hydrophobic spherical powder (i.e., the percentage (Y) may be 100 % by mass).

The "hydrophobic spherical powder" constituting the component (C) has an approximately spherical or spherical shape having a ratio of the long diameter to the short diameter of 1.2 or less and has properties of not being easily dispersed in purified water upon shaking (surface hydrophobicity). The surface hydrophobicity of a powder can be estimated by, for example, dropwise adding acetone to a beaker containing water and the powder with stirring and measuring changes in transmittance of the acetone aqueous solution with a powder wettability tester (WET101-P, manufactured by Rhesca Co., Ltd.). Since the transmittance decreases by dispersion of the powder, the acetone concentration at the point from which the transmittance starts to decrease, i.e., the dispersion starting point, is determined from the slope of the decrease by extrapolation. The surface tension of the acetone aqueous solution can be an index of the critical surface tension of the powder. The hydrophobic powder in the present invention is a powder having surface hydrophobicity in which the approximate value of the critical surface tension determined as described above is about 60 or less, more preferably about 55 or less.

The hydrophobic spherical powder used in the present invention may be an inorganic powder or an organic powder and can be selected from spherical powders made of hydrophobic materials, such as silicone rubber, silicone resin, polyethylene, polymethyl methacrylate, and nylon, or inorganic or organic composite spherical powders surface-hydrophobized with such hydrophobic materials.

Examples of a powder having an average particle size of 1 to 30 µm, which is capable of constituting the component (C), but not corresponding to the hydrophobic spherical powder include powders being easily dispersed in water (i.e., surface hydrophilic powders), such as powders made of silica, starch, cellulose, or the like.

In the present invention, some spherical powders made of a hydrophilic material should be included in the hydrophobic spherical powder as long as surfaces of the powders are hydrophobized to have surface-hydrophobicity according to the definition described above. Conversely, some powders originally made of a hydrophobic material are not included in the hydrophobic spherical powder when the powders have a substantially hydrophilic surface due to the method of production or storage or surface treatment or the like. For example, the spherical powder contained in an aqueous dispersion of a dimethicone/vinyl methicone crosspolymer, available from Dow Corning Toray Co., Ltd. under the product name "BY29-129", is not included in the hydrophobic spherical powder of the present invention.

The content of the (C) powder having an average particle size of 1 to 30 µm in the cosmetic composition of the present invention is 4 to 30 % by mass and preferably 5 to 25 % by mass. The cosmetic composition of the present invention contains a powder having a relatively large average particle size of 1 to 30 µm at a content within the above-mentioned range and thereby provides a correction effect by being applied to the skin and has a good feeling of application.

The cosmetic composition of the present invention contains (D) water and thereby provides a fresh feeling when applied to the skin. The content of (D) water is preferably 30 to 95 % by mass and more preferably 40 to 95 % by mass.

The cosmetic composition of the present invention can further contain (E) a lipophilic nonionic surfactant (hereinafter, also referred to as "lipophilic nonionic surfactant") in addition to the above-mentioned essential components (A) to (D).

The lipophilic nonionic surfactant used in the present invention is a nonionic surfactant that is widely used in cosmetics and the like, and can be selected from those having an HLB value of 6 or less, preferably 5 or less. Examples of the lipophilic nonionic surfactant include, but not limited to, glycerin monostearate, glycerin monostearyl ether, PEG-10 glyceryl triisostearate, PEG-15 hydrogenated castor oil triisostearate, PEG-10 glyceryl trioleate, PEG-10 glyceryl tristearate, PEG-10 trimethylolpropane tristearate, PEG-5 hydrogenated castor oil isostearate, PEG-4 trimethylolpropane distearate, and diisopropyl sebacate.

When the cosmetic composition of the present invention contains the (E) lipophilic nonionic surfactant, the ratio (Z) of the sum of the number of moles of the (A) higher alcohol and the number of moles of the (E) lipophilic nonionic surfactant (total number of moles: A+E) to the number of moles of the (B) hydrophilic surfactant, Z = (A+E)/B, is preferably within a range of 3 to 30.

In the cosmetic composition of the present invention, the (A) higher alcohol forms an associate composed of lamellar bilayer membranes together with the (B) hydrophilic surfactant and preferably with the (E) lipophilic surfactant in the presence of (D) water to take a so-called α-gel structure. The α-gel is of a gel structure that is formed by melting a surfactant and a higher alcohol at high temperature, mixing them with water, and then cooling the mixture; or by melting a higher alcohol at high temperature, mixing it with water in which a surfactant is dissolved, and then cooling the mixture. For details of the α-gel, see, for example, JP-A 2005-132808.

It can be considered that in the cosmetic composition of the present invention, the hydrophobic spherical powder in the component (C) is stably retained in water (aqueous phase) by means of the α-gel structure formed by the components (A), (B), and (D) and preferably with component (E), even if the amounts of the oil and the surfactant are not increased. That is, one of the characteristics of the present invention is that the hydrophobic spherical powder is incorporated in the aqueous phase.

The cosmetic composition of the present invention may contain a water-soluble polymer, such as guar gum, xanthan gum, or polyvinyl alcohol. Although the composition can be further stabilized by incorporating a water-soluble polymer, a larger content of the polymer may cause stickiness. Accordingly, its content should preferably be less than 0.1 % by mass.

The cosmetic composition of the present invention is an aqueous composition which does not contain an oil or may contain an oil in an amount of 30 % by mass or less, 25 % by mass or less, 20 % by mass or less, or 15 % by mass or less.

When the cosmetic composition of the present invention contains an oil, the oil can be selected from oils that are usually used in cosmetics.

Examples of a liquid oil (oil in liquid form at ordinary temperature (about 25°C)) preferably used in the present invention include the followings:
Hydrocarbon oils, such as liquid paraffin, squalane, squalene, paraffin, isoparaffin, and ceresin;
Silicone oils, such as straight-chain (linear) silicone, i.e., dimethylpolysiloxane, methylphenyl polysiloxane, and methyl hydrogen polysiloxane; and cyclic silicone, i.e., octamethylcyclotetrasiloxane, decamethylcyclopentasiloxane, and dodecamethylcyclohexasiloxane;
Liquid oils, such as linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, triglycerin, glycerin trioctanoate, and glycerin triisopalmitate; and
Ester oils, such as cetyl octanoate, hexyl laurate, isopropyl myristate, octyl palmitate, isocetyl stearate, isopropyl isostearate, octyl isopalmitate, isodecyl oleate, glyceryl tri-2-ethylhexanoate, pentaerythritol tetra-2-ethylhexanoate, 2-ethylhexyl succinate, and diethyl sebacate.

The cosmetic of the present invention may contain a solid oil or a semisolid oil that are usually incorporated in cosmetics and the like, in addition to the liquid oil as described above.

The cosmetic composition of the present invention can contain, in addition to the (C) powder having an average particle size of 1 to 30 µm, a powder having an average particle size of less than 1 µm. Such a powder may be any powder that is usually used in cosmetics and may be an inorganic powder or an organic powder and may have any shape, and examples thereof include silica (anhydrous silicate), calcium silicate, magnesium silicate, mica, talc, kaolin, sericite, titanium oxide, zinc oxide, magnesium oxide, zirconium oxide, calcium carbonate, magnesium carbonate, magnesium silicate, barium sulfate, red iron oxide, yellow iron oxide, black iron oxide, carbon black, manganese violet, glass beads, zeolite, pearl pigments (such as red iron oxide-coated mica and titanium oxide-coated mica), and composites thereof.

The cosmetic composition of the present invention can contain various components widely used in the field of cosmetics within a range not impairing the effects of the present invention. Examples of such components include water-soluble active substances, such as vitamin B group, vitamin C and its derivatives, tranexamic acid, pantothenic acid and its derivatives, and vitamins such as biotin; oil-soluble active substances, such as vitamin E and β-carotene; buffer materials, such as arginine, aspartic acid, citric acid, tartaric acid, and lactic acid; chelating agents, such as EDTA; preservatives; and various dyes.

The cosmetic composition of the present invention is particularly suitable for use as an aqueous basic cosmetic having an excellent correction effect, while having a fresh feeling, for example, makeup cosmetics such as foundation and a makeup base or skin care cosmetics such as a milky lotion and an essence liquid (lotion).

### Examples

The present invention will now be described in more detail by examples, but the examples below are representative embodiments of the present invention and do not limit the technical scope of the present invention. The unit of the content is represented by "% by mass".

Essence liquids (lotions) were prepared in accordance with the following method with the following prescriptions shown in Tables 1 to 3. In the tables, Nos. 1 to 3 correspond to component (A), Nos. 7 to 12 correspond to component (B) (among these, Nos. 7 to 10 correspond to nonionic surfactant (B1), and Nos. 11 and 12 correspond to ionic surfactant (B2), and No. 6 corresponds to comparative component (B1') for component (B1)), Nos. 13 to 20 correspond to component (C) (among these, Nos. 13 to 17 correspond to hydrophobic spherical powder (C1), and Nos. 18 to 20 correspond to powder (C2) other than the hydrophobic spherical powders). In addition, Nos. 4 and 5 correspond to lipophilic surfactant (E), and Nos. 21 to 24 correspond to oils.

The resulting essence liquid of each Example was evaluated for the stability of the state of the composition, feelings of use (powdery texture, oily feeling, and sticky feeling), and the appearance of the skin after application based on the criteria shown below.

**[Table 1]**

| Classification | | Example | | | | | | |
|---|---|---|---|---|---|---|---|---|
| | | 1 | 2 | 3 | 4 | 5 | 6 | 7 |
| A | 1. Behenyl alcohol | 0.6 | 0.6 | - | - | 0.5 | 0.5 | - |
| A | 2. Stearyl alcohol | 0.3 | 0.3 | 0.6 | 0.3 | 2 | 0.1 | 0.6 |
| A | 3. Cetyl alcohol | - | - | 1 | 1.5 | - | - | 1 |
| E | 4. Glyceryl monostearate (HLB=4) | - | - | 1 | 0.95 | 1.8 | - | 1 |
| E | 5. Glycerin monostearyl ether (HLB=5) | 0.4 | 0.4 | - | - | - | 0.3 | - |
| B1' | 6. POE (6) sorbitan monostearate (HLB=9.5) | - | - | - | - | - | - | - |
| B1 | 7. POE (20) behenyl ether (HLB=16.5) | 0.6 | 0.6 | - | - | - | 0.3 | - |
| B1 | 8. POE (25) stearyl ether (HLB=14) | - | - | 1 | - | - | - | 1 |
| B1 | 9. POE (60) glycerin monoisostearate (HLB=16) | - | - | - | - | 2.2 | - | - |
| B1 | 10. POE (20) sorbitan monostearate (HLB=14.9) | - | - | - | 1.2 | - | - | - |
| B2 | 11. Sodium stearoyl glutamate | - | - | - | - | - | 0.03 | - |
| B2 | 12. Sodium stearoyl methyl taurate | - | - | - | 0.35 | - | - | - |
| C1 | 13. Vinyl dimethicone/methicone silsesquioxane crosspolymer | - | 6 | - | - | 2 | 13 | - |
| C1 | 14. Polymethylsilsesquioxane | 15 | - | - | - | - | - | - |
| C1 | 15. Dimethicone/vinyl dimethicone crosspolymer | - | - | - | - | 3 | - | - |
| C1 | 16. Polyethylene powder | - | - | - | 7 | - | - | - |
| C1 | 17. Polymethyl methacrylate | - | - | 5 | - | - | - | 5 |
| C2 | 18. Silica | - | 2 | - | 3 | - | - | - |
| C2 | 19. Cellulose powder | - | - | 2 | - | - | - | 2 |
| C2 | 20. Dimethicone/vinyl dimethicone crosspolymer water dispersion | - | - | - | - | - | 13 | - |
| Oil | 21. Pentaerythrityl tetraethylhexanoate | - | 1 | - | 10 | 2 | - | - |
| | 22. Cetyl isooctanoate | 2 | - | - | - | - | 1 | - |
| | 23. Dimethicone | 1 | 2 | 2 | 1 | 5 | 1 | - |
| | 24. Hydrogenated polydecene | 2 | 2 | 5 | 1 | 2 | - | - |
| | 25. Titanium dioxide (average particle size: less than 1 µm) | - | 0.6 | - | - | 0.3 | - | - |
| | 26. Dipropylene glycol | 5 | 5 | 5 | 5 | 5 | 5 | 5 |
| | 27. 1,3-Butylene glycol | 5 | 5 | 5 | 5 | 5 | 5 | 5 |
| | 28. Glycerin | 5 | 3 | - | 5 | 10 | 1 | - |
| | 29. Xanthan gum | - | 0.05 | - | - | 0.07 | - | - |
| | 30. Alcohol | - | 5 | - | - | - | 10 | - |
| | 31. Chelating agent | Appropriate amount | Appropriate amount | Appropriate amount | Appropriate amount | Appropriate amount | Appropriate amount | Appropriate amount |
| | 32. Buffer | Appropriate amount | Appropriate amount | Appropriate amount | Appropriate amount | Appropriate amount | Appropriate amount | Appropriate amount |
| | 33. Preservative | Appropriate amount | Appropriate amount | Appropriate amount | Appropriate amount | Appropriate amount | Appropriate amount | Appropriate amount |
| | 34. Purified water | Balance | Balance | Balance | Balance | Balance | Balance | Balance |
| | | | | | | | | |
| Total amount of powder having an average particle size of 1 to 30 µm [C1+C2] | | 15 | 8 | 7 | 10 | 5 | 21.45 | 7 |
| Percentage of amount of hydrophobic spherical powder [C1/(C1+C2)] × 100 (%) | | 100 | 75 | 71 | 70 | 100 | 61 | 71 |
| Addition phase of hydrophobic spherical powder | | Aqueous phase | Aqueous phase | Aqueous phase | Aqueous phase | Aqueous phase | Aqueous phase | Aqueous phase |
| Molar ratio of hydrophilic surfactant [B1/(B1+B2)] | | 1.00 | 1.00 | 1.00 | 0.53 | 1.00 | 0.78 | 1.00 |
| Molar ratio of lipophilic surfactant, etc. [(A+E)/(B1+B2)] | | 8.3 | 8.3 | 12.8 | 5.7 | 19.0 | 8.7 | 8.7 |
| Stability of state | | A | A | A | A | A | A | A |
| No powdery feeling of use | | B | A | B | B | A | B | B |
| No oily feeling of use | | A | A | B | B | B | A | A |
| No sticky feeling of use | | A | A | B | A | B | A | A |
| Improvement in uniformity of skin appearance after application | | A | A | A | B | B | A | A |

**[Table 2]**

| Classification | | Comparative Example | | | |
|---|---|---|---|---|---|
| | | 1 | 2 | 3 | 4 |
| A | 1. Behenyl alcohol | - | 0.6 | - | - |
| A | 2. Stearyl alcohol | - | 0.3 | 0.3 | 0.3 |
| A | 3. Cetyl alcohol | - | - | 1.5 | 1.5 |
| E | 4. Glyceryl monostearate (HLB=4) | - | - | 0.8 | 0.95 |
| E | 5. Glycerin monostearyl ether (HLB=5) | - | 0.4 | - | - |
| B1' | 6. POE (6) sorbitan monostearate (HLB=9.5) | | - | - | 1.2 |
| B1 | 7. POE (20) behenyl ether (HLB=16.5) | 0.6 | - | - | - |
| B1 | 8. POE (25) stearyl ether (HLB=14) | - | | | |
| B1 | 9. POE (60) glycerin monoisostearate (HLB=16) | - | - | - | - |
| B1 | 10. POE (20) sorbitan monostearate (HLB=14.9) | - | - | 0.7 | - |
| B2 | 11. Sodium stearoyl glutamate | - | - | - | - |
| B2 | 12. Sodium stearoyl methyl taurate | - | 0.21 | 0.25 | 0.35 |
| C1 | 13. Vinyl dimethicone/methicone silsesquioxane crosspolymer | - | 6 | - | - |
| C1 | 14. Polymethylsilsesquioxane | 15 | - | - | - |
| C1 | 15. Dimethicone/vinyl dimethicone crosspolymer | - | - | - | - |
| C1 | 16. Polyethylene powder | - | - | 7 | 7 |
| C1 | 17. Polymethyl methacrylate | - | - | - | - |
| C2 | 18. Silica | - | 2 | 3 | 3 |
| C2 | 19. Cellulose powder | - | - | - | - |
| C2 | 20. Dimethicone/vinyl dimethicone crosspolymer water dispersion | - | - | - | - |
| Oil | 21. Pentaerythrityl tetraethylhexanoate | - | 2 | 10 | 10 |
| | 22. Cetyl isooctanoate | 2 | - | - | - |
| | 23. Dimethicone | 1 | 2 | 1 | 1 |
| | 24. Hydrogenated polydecene | 2 | 2 | 1 | 1 |
| | 25. Titanium dioxide (average particle size: less than 1 µm) | - | 0.5 | - | - |
| | 26. Dipropylene glycol | 5 | 5 | 5 | 5 |
| | 27. 1,3-Butylene glycol | 5 | 5 | 5 | 5 |
| | 28. Glycerin | 5 | 3 | 5 | 5 |
| | 29. Xanthan gum | 0.5 | 0.05 | - | - |
| | 30. Alcohol | - | 5 | - | - |
| | 31. Chelating agent | Appropriate amount | Appropriate amount | Appropriate amount | Appropriate amount |
| | 32. Buffer | Appropriate amount | Appropriate amount | Appropriate amount | Appropriate amount |
| | 33. Preservative | Appropriate amount | Appropriate amount | Appropriate amount | Appropriate amount |
| | 34. Purified water | Balance | Balance | Balance | Balance |
| | | | | | |
| Total amount of powder having an average particle size of 1 to 30 µm [C1+C2] | | 15 | 8 | 10 | 10 |
| Percentage of amount of hydrophobic spherical powder [C1/(C1+C2)] × 100(%) | | 100 | 75 | 70 | 70 |
| Addition phase of hydrophobic spherical powder | | Aqueous phase | Aqueous phase | Aqueous phase | Aqueous phase |
| Molar ratio of hydrophilic surfactant [B1/(B1+B2)] | | 1.00 | 0.00 | 0.48 | 0.00 |
| Molar ratio of lipophilic surfactant, etc. [(A+E)/(B1+B2)] | | 0.0 | 8.4 | 8.5 | 12.2 |
| Stability of state | | B | C | C | C |
| No powdery feeling of use | | C | B | B | - |
| No oily feeling of use | | C | B | B | - |
| No sticky feeling of use | | D | B | B | - |
| Improvement in uniformity of skin appearance after application | | D | D | D | - |

**[Table 3]**

| Classification | | Comparative Example | | | | | | |
|---|---|---|---|---|---|---|---|---|
| | | 5 | 6 | 7 | 8 | 9 | 10 | 11 |
| A | 1. Behenyl alcohol | 0.5 | 0.5 | - | 0.6 | - | 0.6 | 0.6 |
| A | 2. Stearyl alcohol | 2 | 0.1 | 0.3 | 0.3 | 0.6 | 0.3 | 0.3 |
| A | 3. Cetyl alcohol | - | - | 1.5 | - | 1 | - | - |
| E | 4. Glyceryl monostearate (HLB=4) | 1.8 | - | 0.95 | - | 1 | - | - |
| E | 5. Glycerin monostearyl ether (HLB=5) | - | 0.3 | - | 0.4 | - | 0.4 | 0.4 |
| B1' | 6. POE (6) sorbitan monostearate (HLB=9.5) | - | - | - | - | - | - | - |
| B1 | 7. POE (20) behenyl ether (HLB=16.5) | - | 0.3 | - | 0.6 | - | 0.6 | 0.6 |
| B1 | 8. POE (25) stearyl ether (HLB=14) | - | - | - | - | 1 | - | - |
| B1 | 9. POE (60) glycerin monoisostearate (HLB=16) | 2.2 | - | - | - | - | - | - |
| B1 | 10. POE (20) sorbitan monostearate (HLB=14.9) | - | - | 1.2 | - | - | - | - |
| B2 | 11. Sodium stearoyl glutamate | - | 0.03 | - | - | - | - | - |
| B2 | 12. Sodium stearoyl methyl taurate | - | - | 0.1 | - | - | - | - |
| C1 | 13. Vinyl dimethicone/methicone silsesquioxane crosspolymer | - | 22 | - | 3 | - | - | - |
| C1 | 14. Polymethylsilsesquioxane | - | - | - | - | - | 15 | 15 |
| C1 | 15. Dimethicone/vinyl dimethicone crosspolymer | 3 | - | - | - | - | - | - |
| C1 | 16. Polyethylene powder | - | - | - | - | - | - | - |
| C1 | 17. Polymethyl methacrylate | - | - | - | - | - | - | - |
| C2 | 18. Silica | - | - | 10 | 2 | - | - | - |
| C2 | 19. Cellulose powder | - | - | - | 2 | - | - | - |
| C2 | 20. Dimethicone/vinyl dimethicone crosspolymer water dispersion | - | 20 | - | - | 10.8 | - | - |
| Oil | 21. Pentaerythrityl tetraethylhexanoate | 2 | - | 10 | 1 | - | - | - |
| | 22. Cetyl isooctanoate | - | 1 | - | - | - | 2 | 6 |
| | 23. Dimethicone | 5 | 1 | 1 | 2 | 2 | 1 | 2 |
| | 24. Hydrogenated polydecene | 2 | - | 1 | 2 | 5 | 2 | 6 |
| | 25. Titanium dioxide (average particle size: less than 1 µm) | 0.3 | - | - | 0.6 | - | - | - |
| | 26. Dipropylene glycol | 5 | 5 | 5 | 5 | 5 | 5 | 5 |
| | 27. 1,3-Butylene glycol | 5 | 5 | 5 | 5 | 5 | 5 | 5 |
| | 28. Glycerin | 10 | 1 | 5 | 3 | - | 5 | 5 |
| | 29. Xanthan gum | 0.07 | - | - | 0.05 | - | - | - |
| | 30. Alcohol | - | 10 | - | 5 | - | - | - |
| | 31. Chelating agent | Appropriate amount | Appropriate amount | Appropriate amount | Appropriate amount | Appropriate amount | Appropriate amount | Appropriate amount |
| | 32. Buffer | Appropriate amount | Appropriate amount | Appropriate amount | Appropriate amount | Appropriate amount | Appropriate amount | Appropriate amount |
| | 33. Preservative | Appropriate amount | Appropriate amount | Appropriate amount | Appropriate amount | Appropriate amount | Appropriate amount | Appropriate amount |
| | 34. Purified water | Balance | Balance | Balance | Balance | Balance | Balance | Balance |
| | | | | | | | | |
| Total amount of powder having an average particle size of 1 to 30 µm [C1+C2] | | 3 | 35 | 10 | 7 | 7.02 | 15 | 15 |
| Percentage of amount of hydrophobic spherical powder [C1/(C1+C2)] × 100 (%) | | 100 | 63 | 0 | 43 | 0 | 100 | 100 |
| Addition phase of hydrophobic spherical powder | | Aqueous phase | Aqueous phase | - | Aqueous phase | Aqueous phase | Oil phase | Oil phase |
| Molar ratio of hydrophilic surfactant [B1/(B1+B2)] | | 1.00 | 0.78 | 0.53 | 1.00 | 1.00 | 1.00 | 1.00 |
| Molar ratio of lipophilic surfactant, etc. [(A+E)/(B1+B2)] | | 19.0 | 8.7 | 5.7 | 8.3 | 12.8 | 8.3 | 8.3 |
| Stability of state | | A | B | A | A | A | C | B |
| No powdery feeling of use | | B | D | C | C | B | - | B |
| No oily feeling of use | | C | B | B | B | B | - | D |
| No sticky feeling of use | | B | B | B | B | C | - | C |
| Improvement in uniformity of skin appearance after application | | D | C | D | D | C | - | D |

Method of production in Examples and Comparative Examples

### (1) Examples 1, 3, 4, 6, and 7 and Comparative Examples 1, 3, 6, and 7

A mixture in which components 1 to 10 and 21 to 24 were heated to 70°C and were uniformly melted and mixed was added to a mixture in which components 11, 12, and 26 to 34 were dissolved, mixed, and heated to 70°C, followed by emulsification with a stirrer (T.K. Robomix, manufactured by Primix Corporation) at 7000 rpm. Components 13 to 20 were added to the emulsion, and the mixture was dispersed again with a stirrer, followed by cooling to 35°C to prepare an essence liquid.

### (2) Examples 2 and 5 and Comparative Examples 2, 4, 5, and 8

A mixture in which components 1 to 10 and 21 to 24 were heated to 70°C and were uniformly melted and mixed was added to a mixture in which components 11, 12, 26 to 30, components 32 and 33, and component 34 except for 10 wt% were dissolved, mixed, and heated to 70°C, followed by emulsification with a stirrer at 7000 rpm. A mixture in which components 25 and 31 and 10 wt% of component 34 were mixed and dispersed with a stirrer was added to the emulsion. Components 13 to 20 were further added to the mixture, followed by dispersion again with a stirrer. The dispersion was cooled to 35°C to prepare an essence liquid.

### (3) Comparative Examples 10 and 11

Component 14 was added to a mixture in which components 1 to 10 and 21 to 24 were heated to 70°C and were uniformly melted and mixed, followed by dispersion with a stirrer. The dispersion was added to a mixture in which components 11, 12, and 26 to 34 were dissolved, mixed, heated to 70°C, followed by emulsification with a stirrer at 7000 rpm. The emulsion was cooled to 35°C to prepare an essence liquid.

### Method for evaluation and evaluation criteria

### • Stability of the state

The states of samples were evaluated with the below-listed criteria by preparing samples according to the methods of production as described above, dropping an appropriate amount of each sample on a slide glass, covering it with a cover glass, and visually observing the state of the sample, (1) after leaving the sample for one day from the preparation (on the next day); (2) after placing the sample on the next day in a 50-cc glass tube until the glass tube was filled halfway and subsequently rotating the glass tube in the inverted and the normal direction at 45 rpm for 4 hours; and (3) after storage for one month from the preparation.
A: Aggregates were not observed under all the above-mentioned three conditions (1) to (3);
B: The sample (standing) on the next day of the preparation (1) was homogeneous, but aggregates were observed under other two conditions (2) and (3); and
C: Aggregates were observed in the sample (standing) on the next day of the preparation (1) and also under other two conditions (2) and (3).

### • Feeling of use (powdery texture, oily feeling, and sticky feeling)

Ten sensory evaluation panelists applied each sample to the skin and evaluated the feeling immediately after the application for three items, "powdery texture, oily feeling, and sticky feeling" each by four levels: "Presence: 1 point", "Slight presence: 2 points, "Not much presence: 3 points", and Absence: 4 points". The samples were each evaluated by the average point of each item as follows:
A: 3.26 to 4 points,
B: 2.51 to 3.25 points,
C: 1.76 to 2.5 points, and
D: 1 to 1.75 points.

### • Effect of improving in uniformity of skin appearance after application

Each sample was applied to five female subjects in their thirties and was evaluated by five evaluation panelists for the uniformity of the skin appearance after the application by three levels: "Improved: 3 points", "Slightly improved: 2 points", and "Not improved: 1 point". The scores of the five subjects by the five evaluation panelists were averaged, and the effect of each sample was determined as follows:
A: 2.6 to 3 points,
B: 2.1 to 2.5 points,
C: 1.6 to 2 points, and
D: 1 to 1.5 points.

As obvious from the results shown in Table 1, Examples 1 to 7 satisfying the requirements of the present invention were all satisfactory in every evaluation item.

The results shown in Table 2 demonstrate the following facts.

Comparative Example 1 is an example in which a powder was dispersed with only a surfactant, without using a gel formed by a higher alcohol and a surfactant, and had a sticky feeling of use. In addition, since there was no thickening effect by a gel formed by a higher alcohol and a surfactant, a thickening agent was added for maintaining a uniform dispersion state of the powder. However, the thickening agent formed a composite with the powder when applied to the skin. Consequently, the powder did not uniformly spread, and there was no effect of improving uniformity of skin appearance.

In Comparative Example 2, only a surfactant of ionic type was used as the hydrophilic surfactant. In Comparative Example 3, the molar concentration of the ionic hydrophilic surfactant was higher than that of the nonionic one. In both of these Comparative Examples, thickening effects were insufficient for maintaining the state stable, and the dispersion state of the powder was slightly poor to show a low effect of improving uniformity of skin appearance.

In Comparative Example 4, the hydrophilic nonionic surfactant (HLB = 14.5) in Example 4 was replaced with a nonionic surfactant having an HLB (9.5) of less than 10, and a stable formulation could not be prepared.

The results shown in Table 3 demonstrate the following facts.

In Comparative Example 5, the amount of the powder was small, and the effect of improving uniformity of skin appearance was low. In Comparative Example 6, the amount of the powder was large, in addition to a powdery feeling (texture), powdery appearance of the skin after the application was also conspicuous.

In Comparative Example 7, the powders were all hydrophilic. In Comparative Example 8, the percentage of the hydrophobic spherical powder in all powders was 43%. In both of these Comparative Examples, a powdery feeling (texture) was observed, and the affinity of the powder to the skin was low, and the effect of improving uniformity of skin appearance was poor.

In Comparative Example 9, a raw material provided as a water dispersion of a hydrophobic spherical powder was used. The raw material also contained a surfactant and a thickening agent for stably dispersing a powder, and sticky feeling was observed similar to Comparative Example 3. In addition, the powder surface was substantially hydrophilic, and the effect of improving uniformity of skin appearance was low similar to Comparative Examples 7 and 8.

In Comparative Examples 10 and 11, a hydrophobic spherical powder was dispersed in the oil phase. The amount of oil in Comparative Example 10 was insufficient for dispersing the powder, and therefore the dispersion did not become uniform, resulting in a poor state immediately after the production. In Comparative Example 11, sufficient amount of oils for dispersing the powder were used, but it caused oily feeling and resulted in poor adhesion of the powder to the skin.

Other examples of the prescriptions of the cosmetic compositions of the present invention are shown below.

### Prescription example 1:

| Cream | Content (% by mass) |
|---|---|
| 1 Behenyl alcohol | 2 |
| 2 Stearyl alcohol | 2 |
| 3 Glycerin monostearyl ether | 2 |
| 4 POE (20) behenyl ether | 1.5 |
| 5 Vinyl dimethicone/methicone silsesquioxane crosspolymer | 10 |
| 6 Silica | 2 |
| 7 Pentaerythrityl tetraethylhexanoate | 5 |
| 8 Dimethicone | 3 |
| 9 Hydrogenated polydecene | 2 |
| 10 Titanium dioxide | 0.1 |
| 11 1,3-Butylene glycol | 10 |
| 12 Glycerin | 10 |
| 13 Ascorbyl glucoside | 2 |
| 14 Acrylates/C10-30 alkyl acrylate copolymer (*16) | 0.05 |
| 15 Xanthan gum | 0.1 |
| 16 Alcohol | 3 |
| 17 Chelating agent | appropriate amount |
| 18 Buffer | appropriate amount |
| 19 Preservative | appropriate amount |
| 20 Purified water | balance |

| | |
|---|---|
| *16: PEMULEN TR-2 (Lubrizol Advanced Materials, Inc.) | |

### Method of production

An aqueous phase in which components 10 to 20 were uniformly mixed and dissolved was heated to 70°C, and an oil phase in which components 1 to 4 and 7 to 9 were melted by heating to 75°C was added to the aqueous phase. Components 5 and 6 were added to the mixture and were uniformly dispersed therein. The dispersion was cooled to 30°C while stirring to prepare cream.

### Prescription example 2:

| Essence liquid | Content (% by mass) |
|---|---|
| 1 Cetearyl alcohol | 0.4 |
| 2 Glycerin monostearyl ether | 0.3 |
| 3 POE (60) hydrogenated castor oil (*17) | 0.5 |
| 4 Vinyl dimethicone/methicone silsesquioxane crosspolymer (*18) | 15 |
| 5 Dimethicone/vinyl dimethicone crosspolymer water dispersion | 20 |
| 6 Isohexadecane | 10 |
| 7 Hydrogenated jojoba oil | 2 |
| 8 Dipropylene glycol | 5 |
| 9 Glycerin | 5 |
| 10 Ammonium acryloyldimethyltaurate/beheneth-25 methacrylate crosspolymer (*19) | 0.3 |
| 11 Chelating agent | appropriate amount |
| 12 Buffer | appropriate amount |
| 13 Preservative | appropriate amount |
| 14 Purified water | balance |

| | |
|---|---|
| *17: Nikkol HCO-60 (Nikko Chemicals Co., Ltd.) *18: KSP102 (Shin-Etsu Chemical Co., Ltd.) *19: ARISTOFLEX HMB (Clariant (Japan) K.K.) | |

### Method of production

An aqueous phase in which components 8 to 14 were mixed was heated to 70°C. An oil phase was prepared by adding component 6 to components 1 to 3 and 7 melted by heating to 70°C. The oil phase was added to the aqueous phase, and the mixture was emulsified and dispersed with a homomixer, followed by cooling to 35°C. Components 4 and 5 were added thereto, followed by dispersion with a homomixer to prepare an essence liquid.

### Prescription example 3:

| Foundation | Content (% by mass) |
|---|---|
| 1 Behenyl alcohol | 0.4 |
| 2 Stearyl alcohol | 0.1 |
| 3 Glyceryl monostearate | 0.1 |
| 4 POE (30) Behenyl ether | 0.3 |
| 5 Dimethicone/vinyl dimethicone crosspolymer | 5 |
| 6 Polymethyl methacrylate | 5 |
| 7 Decamethylsiloxane | 6 |
| 8 Methylpolysiloxane | 3 |
| 9 Di-2-ethylhexyl succinate | 2 |
| 10 Ethylhexyl methoxycinnamate | 5 |
| 11 Octocrylene | 2 |
| 12 Hydrophobized (*21) titanium dioxide | 5 |
| 13 Hydrophobized (*21) red iron oxide | 0.1 |
| 14 Hydrophobized (*21) yellow iron oxide | 0.5 |
| 15 Hydrophobized (*21) black iron oxide | 0.01 |
| 16 Aminoethylaminopropylmethylsiloxane/dimethylsiloxane copolymer (*22) | 1 |
| 17 1,3-Butylene glycol | 3 |
| 18 Glycerin | 1 |
| 19 Xanthan gum | 0.1 |
| 20 Hydroxyethyl acrylate/Na acryloyldimethyltaurate copolymer (*23) | 0.5 |
| 21 Alcohol | 5 |
| 22 Chelating agent | appropriate amount |
| 23 Buffer | appropriate amount |
| 24 Preservative | appropriate amount |
| 25 Purified water | balance |

| | |
|---|---|
| *21: Tetradecene, tetrahydrotetramethylcyclotetrasiloxane treatment *22: KF-8004 (Shin-Etsu Chemical Co., Ltd.) *23: SIMULGEL NS (SEPPIC S.A.) | |

### Method of production

An aqueous phase in which components 17 to 25 were mixed was heated to 70°C. An oil phase (1) in which components 1 to 4 and 9 to 11 were heated to 70°C was added to the aqueous phase, and the mixture was emulsified with a homomixer and was then cooled to 30°C. An oil phase (2) in which components 12 to 16 were added to and dispersed in components 7 and 8 was added to the emulsion, and the mixture was emulsified and dispersed with a homomixer again. Components 5 and 6 were added thereto, followed by dispersion again to prepare foundation.

## Claims

1. A cosmetic composition comprising:
(A) at least one higher alcohol having a saturated straight-chain alkyl chain;
(B) a hydrophilic surfactant including a nonionic surfactant;
(C) 4 to 30 % by mass of a powder having an average particle size of 1 to 30 µm; and
(D) water,
wherein a ratio of the number of moles of the hydrophilic nonionic surfactant to the total number of moles of the (B) hydrophilic surfactant is at least 0.5;
wherein a percentage of the content of a hydrophobic spherical powder in the total content of the (C) powder having an average particle size of 1 to 30 µm is at least 50 % by mass, and
wherein the hydrophobic spherical powder is contained in an aqueous phase.

2. The cosmetic composition according to Claim 1, further comprising:
(E) a lipophilic nonionic surfactant, wherein:
Providing the number of moles of the (A) higher alcohol is (A), the number of moles of the lipophilic nonionic surfactant is (E), and the number of moles of the hydrophilic surfactant is (B), the ratio expressed by a formula [(A+E)/B] is within a range of 3 to 30.

3. The cosmetic composition according to Claim 1 or 2, wherein:
a content of the (C) powder having an average particle size of 1 to 30 µm is in the range of 5 to 25 % by mass.

4. The cosmetic composition according to any one of Claims 1 to 3, further comprising:
a water-soluble polymer in an amount of at most 0.1 % by mass.

5. The cosmetic composition according to any one of Claims 1 to 4, wherein:
the nonionic surfactant included in the (B) hydrophilic surfactant is a polyalkylene glycol saturated straight-chain alkyl ether.

6. The cosmetic composition according to any one of Claims 1 to 5, wherein:
the hydrophobic spherical powder contained in the (C) powder having an average particle size of 1 to 30 µm is at least one powder selected from a group consisting of silicone rubber, silicone resin, and composite a spherical powder thereof.
